# EUROPÄISCHE PATENTANMELDUNG

(11) **EP 4 342 512 A1**
(43) Veröffentlichungstag der Anmeldung: **27.03.2024**
(21) Anmeldenummer: 22196458.8
(22) Anmeldetag: 20.09.2022
(51) Int. Cl.: A61M 16/00

(54) **VORRICHTUNG ZUM INHALIEREN EINES GASGEMISCHES MIT ZUMINDEST EINEM ANTEIL OZON**

(71) Anmelder: Polanski, Slava, 56745 Rieden (DE)
(72) Erfinder: Polanski, Slava, 56745 Rieden (DE)
(74) Vertreter: Bauer, Dirk

(57) **Zusammenfassung**

Die Erfindung betrifft eine Vorrichtung (1) zum Inhalieren eines Gasgemisches (2) mit zumindest einem Anteil Ozon (3), umfassend ein Inhalationsteil (4) und einen Behälter (5) zum Speichern von Ozon (3) oder des Gasgemisches (2). Außerdem umfasst die Vorrichtung (1) ein Leitungselement (6), mittels welchem der Behälter (5) fluidleitend mit dem Inhalationsteil (4) verbunden ist. Hierbei weist das Inhalationsteil (4) wenigstens eine Öffnung (7) auf, aus der das Gasgemisch (2) ausströmen kann. Erfindungsgemäß ist vorgesehen, dass das Gasgemisch (2) in dem Behälter (5) gasförmig vorliegt.

## Beschreibung

Die Erfindung betrifft eine Vorrichtung zum Inhalieren eines Gasgemisches mit zumindest einem Anteil Ozon gemäß dem Oberbegriff des Anspruchs 1.

Die Vorrichtung zum Inhalieren eines Gasgemisches mit zumindest einem Anteil Ozon umfasst ein Inhalationsteil und einen Behälter zum Speichern von Ozon oder des Gasgemisches. Außerdem weist die Vorrichtung ein Leitungselement auf, mittels welchem der Behälter fluidleitend mit dem Inhalationsteil verbunden ist. Hierbei weist das Inhalationsteil wenigstens eine Öffnung auf, aus der das Gasgemisch ausströmen kann.

### Stand der Technik

Es gilt als wissenschaftlich belegt, dass Ozon eine keimabtötende und desinfizierende Wirkung hat. Durch Ozon können also Krankheiterreger, beispielsweise Viren, Bakterien und Pilze abgetötet oder zumindest an ihrem Wachstem gehindert werden. Mit anderen Worten ist Ozon viruzid, fungizid und bakterizid. Daher hat sich ein Einsatz von Ozon schon in der Vergangenheit zur Virusbekämpfung als hilfreich erwiesen, zum Beispiel bei dem SARS-Virus von 2002.

Vorrichtungen zum Inhalieren eines Gasgemisches, das zumindest einen Anteil Ozon oder Sauerstoff umfasst, sind dem Stand der Technik in vielfältiger Ausgestaltung als bekannt zu entnehmen. Hierzu sei beispielsweise auf das von der ACP Japan Co. Ltd. angemeldete europäische Patent EP 2 345 443 A1 hingewiesen, dem ein Gasnebelinhalator als bekannt zu entnehmen ist. Der darin beschriebene Gasnebelinhalator umfasst einen Behälter, der das gespeicherte Gasgemisch enthält und mit einem Gasnebelgenerator verbunden ist, über den das Gasgemisch durch Zerstäuben und Lösen zu einem Inhalationsteil geführt wird, mittels welchem es über die oberen und unteren Atemwege aufgenommen werden kann. Der Gasnebelgenerator ist hierbei in einer Flüssigkeit angeordnet, wobei die Zerstäubung durch eine Düse, durch die das Gasgemisch mit hoher Geschwindigkeit austritt, erreicht wird. Mittels einer Platte wird der Gasnebel von der Flüssigkeit getrennt. Zur Erzeugung des Gasnebels wird das Gasgemisch unter Druck aus dem Behälter über die Düse in den Gasnebelgenerator geleitet. Dies erzeugt an einem Austritt der Düse einen Unterdruck, wodurch die Flüssigkeit, welche ebenfalls mittels Leitungen mit dem Austritt der Düse verbunden ist, in Richtung des Austrittes der Düse gesaugt wird. Die austretende Flüssigkeit prallt gegen eine gegenüber dem Austritt der Düse befindliche Ablenkplatte, wodurch die Flüssigkeit zerstäubt wird. Der erzeugte Gasnebel wird über eine Öffnung in den von dem Inhalationsteil und einer Person, der das Inhalationsteil angelegt ist, eingeschlossenen Raum geleitet. Als zu verwendende Flüssigkeit wird beispielsweise Ozonwasser aufgeführt.

Dem Stand der Technik sind außerdem Weiterentwicklungen der vorgenannten Patentanmeldung als bekannt zu entnehmen. Hierbei offenbart beispielsweise die europäische Patentanmeldung EP 2 679 264 A1 eine Weiterentwicklung, bei der ein Gehäuse des Gasnebelinhalators weiterentwickelt wurde. Dazu ist gemäß der EP 2 679 264 A1 eine äußere Leitung in einen Hauptkörper, in dem auch der Gasnebelgenerator angeordnet ist, integriert. Dementsprechend befindet sich eine Öffnung zur Zufuhr von Frischluft in einer Wand des Gasnebelgenerators oder in dem Inhalationsteil.

Nachteilig bei dem vorgenannten Stand der Technik ist, dass das Gasgemisch nicht in gasförmiger Form gespeichert wird und somit ein aufwendiger und kostenintensiver Mechanismus zur Zerstäubung notwendig ist.

### Aufgabe

Es ist daher Aufgabe der vorliegenden Erfindung, eine Vorrichtung zum Inhalieren eines Gasgemisches, das zumindest einen Anteil Ozon umfasst, derart weiterzuentwickeln, dass die Nachteile des Stands der Technik beseitigt werden.

### Lösung

Die vorgenannte Aufgabe wird erfindungsgemäß durch eine Vorrichtung gemäß dem Anspruch 1 gelöst. Vorteilhafte Ausgestaltungen sind Gegenstand der Unteransprüche 2 bis 11.

Hierzu ist es erfindungsgemäß vorgesehen, dass das Gasgemisch in dem Behälter gasförmig vorliegt.

Die Erfindung hat viele Vorteile.

Einer Person, die das Gasgemisch inhalieren soll, wird das Gasgemisch über ein Inhalationsteil zum Inhalieren bereitgestellt. Das Inhalieren kann über die Atemwege der Person, beispielsweise den Mund oder die Nase, erfolgen. Mit anderen Worten inhaliert die Person das Gasgemisch mittels des Mundes oder/und der Nase, sodass das Gasgemisch in den Körper der Person einströmen kann. Das Inhalationsteil ist daher derart beschaffen, dass es eine Öffnung aufweist, aus der das Gasgemisch ausströmen und somit von der Person inhaliert werden kann.

Das Inhalationsteil wird aus einem Behälter gespeist, in dem das Gasgemisch erfindungsgemäß gasförmig vorliegt. Dazu ist der Behälter mit dem Inhalationsteil mittels eines Leitungselements verbunden. Mit anderen Worten strömt das Gasgemisch aus dem Behälter durch das Leitungselement in das Inhalationsteil, von wo aus es von der Person inhaliert werden kann. Hierdurch ergibt sich der Vorteil, dass das Gasgemisch schon gasförmig vorliegt, nicht also erst gasförmig gemacht werden muss. Vorteilhafterweise ergibt sich somit ein besonders einfacher und kostengünstiger Betrieb.

In einer besonders vorteilhaften Ausgestaltung ist eine Steuereinrichtung vorgesehen, mittels derer eine Zusammensetzung des Gasgemisches, insbesondere dessen Anteil an Ozon, durch Mischung mit einem weiteren Gas oder Gasgemisch eingestellt werden kann. Hierzu kann beispielsweise ein weiterer Behälter vorgesehen sein, in dem ein von dem im ersten Behälter befindliches unterschiedliches Gas oder Gasgemisch gespeichert ist. Folglich werden die Gase beziehungsweise Gasgemische der jeweiligen Behälter zusammengeführt, sodass die Zusammensetzung des dadurch entstehenden (sekundären) Gasgemisches einstellbar ist. Hierzu wird eine Zuführung der jeweiligen Gase beziehungsweise Gasgemische mittels der Steuereinrichtung derart eingestellt, dass die gewünschte Zusammensetzung des durch Mischung der jeweiligen Gase beziehungsweise Gasgemische entstehenden Gasgemisches erzielt wird. Vorteilhafterweise ergibt sich hierdurch ein besonders bedarfsgerechter und individueller Betrieb.

Die Erfindung weiter ausgestaltend ist eine Ventileinrichtung vorgesehen, mittels welcher ein Durchfluss des Gasgemisches unterbrechbar und/oder regulierbar ist. Da die Person zyklisch ein- und ausatmet, kann es vorteilhaft sein, das Gasgemisch nicht kontinuierlich bereitzustellen. Mit anderen Worten ist es für die Person unangenehm und unter Verbrauchsaspekten auch unnötig, wenn ihr das Gasgemisch entgegenströmt, während sie gerade ausatmet. Hierdurch wird ein Ausatmen sogar behindert. Mittels des Ventils kann der Durchfluss unterbrochen und/oder reguliert werden, dass derartige Situation vermieden werden. Vorteilhafterweise ergibt sich hierdurch ein besonders komfortabler und Gasgemisch sparender, d.h. ressourceneffizienter, Betrieb.

Vorteilhafterweise ist vorgesehen, dass die Ventileinrichtung mittels der Steuereinrichtung steuerbar ist. Es kann beispielsweise vorgesehen sein, die Ventileinrichtung in Abhängigkeit von einem Atemzyklus der Person zu steuern. In diesem Zusammenhang kann es beispielsweise vorgesehen sein, dass das Ventil geschlossen wird beziehungsweise ist, also kein Gasgemisch zum Inhalieren bereitstellt, wenn die Person ausatmet, und geöffnet wird beziehungsweise ist, wenn die Person einatmet. Hierdurch ist ein Widerstand, den die Person zum Inhalieren des Gasgemisches, insbesondere mittels deren Lunge, überwinden muss, besonders gering. Vorteilhafterweise ist der Betrieb daher besonders mühelos und schonend möglich.

Ein weiterer Vorteil ergibt sich dadurch, dass das Inhalationsteil eine Maske aus einem fluidundurchlässigen Material ist, welche zumindest einen Mund- und Nasenbereich einer die Maske tragenden Person umschließt und an den Körper der Person im Kopf- und Halsbereich vorzugsweise randseitig umlaufend abdichtend anschließt. Durch die Maske ergibt sich somit ein Raum, der von der Maske und einem Körperbereich, an dem die Maske an der Person anliegt, eingeschlossen wird. Vorzugsweise liegt die Maske entlang ihrer Ränder derart an der Person an, dass ein Luftaustausch mit einer Umgebung vermieden wird. Mit anderen Worten wird dem Raum lediglich das Gasgemisch zugeführt. Insbesondere tritt in den Raum keine Umgebungsluft ein. Vorteilhafterweise ergibt sich dadurch ein besonders schadstoffarmer und gesunder Betrieb, da jegliche Krankheitserreger oder sonstige Schadstoffe, welche beispielsweise in der Umgebungsluft enthalten sind, nicht inhaliert werden.

Gemäß einer weiteren besonders vorteilhaften Ausgestaltung ist vorgesehen, dass das Inhalationsteil eine Maske aus einem fluiddurchlässigen Material ist. Gemäß dieser Ausgestaltung ist es vorgesehen, dass die Person (auch) die Umgebungsluft inhalieren kann, und zwar mittels des fluiddurchlässigen Materials. Alternativ oder zusätzlich kann es vorgesehen sein, dass das fluiddurchlässige Material eine Filterwirkung aufweist oder in Kombination mit einem filternden Material vorgesehen ist. Dadurch, dass das Material fluiddurchlässig ist, muss ein besonders geringer Widerstand beim Atemzyklus überwunden werden. Mit anderen Worten ist es bei dem derartig beschaffenen Material für die Person einfacher, ein- beziehungsweise auszuatmen. Hierdurch ergibt sich der Vorteil, dass die Anwendung für die Person besonders anstrengungsarm ist.

Vorteilhafterweise ist vorgesehen, dass das Inhalationsteil einen Lufteinlass aufweist. Mittels dieses Lufteinlasses kann beispielsweise Umgebungsluft einströmen. Mit anderen Worten wird in dem Inhalationsteil das Gasgemisch mit der Umgebungsluft gemischt. Die Person inhaliert somit eine Mischung aus der Umgebungsluft und dem Gasgemisch. Da die Person folglich immer auch einen Anteil an im Wesentlichen unbehandelter Umgebungsluft inhaliert, ergibt sich somit ein besonders natürlicher und intuitiver Betrieb.

In einer weiteren besonders vorteilhaften Ausgestaltung ist ein Mischraum zum Mischen des aus dem Behälter austretenden Ozons oder Gasgemisches mit dem weiteren Gas oder Gasgemisch vorgesehen, wobei der Mischraum an dem Behälter oder in dem Leitungselement oder in dem Inhalationsteil angeordnet ist. Innerhalb des Mischraums können sich aufgrund dessen Volumens die Gase beziehungsweise Gasgemische besonders gut ausbreiten und somit mischen. Dies hat zur Folge, dass ein besonders homogenes Gasgemisch bereitgestellt werden kann, wodurch vorteilhafterweise ein besonders qualitativ hochwertiger Betrieb möglich ist.

Weiterhin vorteilhafterweise ist ein Drucksensor vorgesehen, mittels welchem ein Druck des Gasgemisches in dem Inhalationsteil und/oder dem mindestens einen Behälter ermittelbar ist. Ein zu hoher oder niedriger Druck des Gasgemisches kann beispielsweise für die Lunge beim Atemzyklus gefährlich werden. Liegt ein zu hoher oder niedriger Druck des Gasgemisches vor, kann die Lunge überbeansprucht und somit geschädigt werden. Mittels des Drucksensors kann der Druck ermittelt werden, wodurch sich vorteilhafterweise ein besonders sicherer Betrieb realisieren lässt.

Gemäß einer weiteren besonders vorteilhaften Ausgestaltung ist ein Konzentrationssensor vorgesehen, mittels welchem die Zusammensetzung des Gasgemisches, insbesondere in dem Inhalationsteil, ermittelbar ist. Mit anderen Worten können mittels des Konzentrationssensors beispielsweise für die Person schädliche oder gefährliche Konzentration von Schadstoffen oder einzelnen Bestandteilen des Gasgemisches ermittelt werden. Hierdurch ergibt sich vorteilhafterweise ein besonders sicherer und gesundheitsschonender Betrieb.

Vorteilhafterweise ist vorgesehen, dass ein von dem Konzentrationssensor ermittelter Wert mittels einer Anzeigeeinrichtung darstellbar ist. Hierdurch kann die Person auf den ermittelten Wert aufmerksam gemacht werden und somit aktuell vorliegenden Konzentrationen des Gasgemisches in Beobachtung halten. Hierdurch ergibt sich ein besonders verständlicher, ansprechender und sicherer Betrieb.

### Ausführungsbeispiele

Die Erfindung ist nachstehend anhand eines Ausführungsbeispiels, das in den Figuren dargestellt ist, näher erläutert. Es zeigt:
- Fig. 1:: eine schematische Gesamtansicht einer erfindungsgemäßen Vorrichtung; und
- Fig. 2:: eine schematische Gesamtansicht der erfindungsgemäßen Vorrichtung gemäß einer weiteren Ausgestaltung.

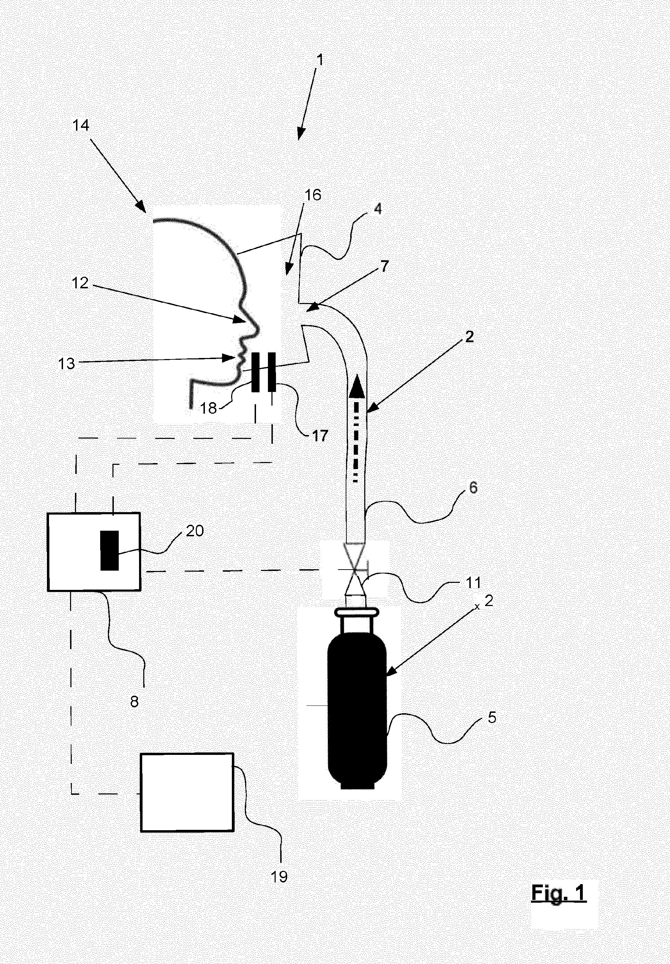

Figur 1 zeigt in einer schematischen Gesamtansicht eine erfindungsgemäße Vorrichtung 1. Vorliegend umfasst die Vorrichtung 1 ein Inhalationsteil 4 sowie einen Behälter 5. In dem Behälter 5 ist beispielsweise reines Ozon 3 oder ein Gasgemisch 2 gespeichert, das zumindest einen Anteil Ozon 3 enthält. Eine weitere Gaskomponente des Gemischs kann beispielsweise von Sauerstoff und/oder Luft und/oder einem Gemisch aus Ozon und Luftgebildet werden. Außerdem ist das Ozon 3 beziehungsweise das Gasgemisch 2 in dem Behälter 5 gasförmig gespeichert. Weiterhin umfasst die Vorrichtung 1 ein Leitungselement 6, mittels welchem der Behälter 5 fluidleitend mit dem Inhalationsteil 4 verbunden ist. Bei diesem Leitungselement 6 kann es sich beispielsweise um einen flexiblen Schlauch oder ein Rohr handeln. Zudem weist das Inhalationsteil 4 eine Öffnung 7 auf, aus der das Ozon 3 beziehungsweise das Gasgemisch 2 ausströmen kann.

Wie Figur 1 zeigt, kann das Inhalationsteil 4 beispielsweise in Form einer Maske ausgestaltet sein, die einer Person 14 im Gesicht aufgesetzt werden kann. Hierzu umschließt das Inhalationsteil 4 zumindest einen Mundbereich 12 sowie einen Nasenbereich 13 der Person 14. Mit anderen Worten liegt das Inhalationsteil 4 an seinen Rändern umlaufend im Wesentlichen dicht auf dem Gesicht der Person 14 an. Vorliegend umfasst die Vorrichtung 1 einen Mischraum 16, der gemäß Figur 1 innerhalb des Inhalationsteils 4 angeordnet ist.

Außerdem umfasst die Vorrichtung 1 vorliegend eine Ventileinrichtung 11, mittels derer ein Durchfluss des Ozons 3 beziehungsweise des Gasgemisches 2 einstellbar ist. Wie Figur 1 zeigt, ist die Ventileinrichtung 11 in Flussrichtung des Ozons 3 beziehungsweise des Gasgemisches 2 dem Behälter 5 nachgeschaltet. Die Ventileinrichtung 11 kann beispielsweise in das Leitungselement 6 integriert sein.

Das Ozon 3 beziehungsweise das Gasgemisch 2 strömt folglich aus dem Behälter 5 durch das Leitungselement 6 in das Inhalationsteil 4, wo es von der Person 14 inhaliert werden kann.

Die Vorrichtung 1 umfasst weiterhin einen Drucksensor 17 sowie einen Konzentrationssensor 18, die im Inhalationsteil 4 angeordnet sind, wie Figur 1 deutlich zeigt. Mittels des Drucksensors 17 kann ein Druck des Ozons 3 beziehungsweise des Gasgemisches 2 ermittelt werden. Entsprechend kann mittels des Konzentrationssensors 18 eine Konzentration des Ozons 3 beziehungsweise von Bestandteilen des Gasgemisches 2 ermittelt werden.

Gemäß Figur 1 umfasst die Vorrichtung 1 außerdem eine Steuereinrichtung 8. Es kann gemäß der in Figur 1 gezeigten Ausgestaltung vorgesehen sein, dass die Steuereinrichtung 8 eine Speichereinrichtung 20 umfasst, mittels welcher Werte gespeichert werden können. Die Steuereinrichtung 8 ist vorliegend signalübertragend, insbesondere leitungslos, mit der Ventileinrichtung 11 verbunden. Außerdem ist die Steuereinrichtung 8 mit dem Drucksensor 17 sowie dem Konzentrationssensor 18 signalübertragend, insbesondere leitungslos, verbunden. Infolgedessen kann mittels der Steuereinrichtung 8 beispielsweise der Durchfluss durch Regeln der Ventileinrichtung 11 derart eingestellt werden, dass der mittels des Drucksensors 17 gemessene Druck beziehungsweise die mittels des Konzentrationssensors 18 gemessene Konzentration einem Sollwert entspricht. Unter medizinischen Aspekten sollte die Konzentration des Ozons in dem von der Person inhalierten Gasgemisch 0,03 g/dm³ bis 0,04 g/dm³ betragen.

Außerdem umfasst die Vorrichtung 1 eine Anzeigeeinrichtung 19, mittels welcher von der Steuereinrichtung 8 ermittelte Werte angezeigt werden können. Die Anzeigeeinrichtung 19 kann beispielsweise ein Display, insbesondere ein LCD-Display, sein. Weiterhin denkbar ist, dass die Anzeigeeinrichtung 19 einen Lautsprecher umfasst, mittels dessen bei Überschreiten oder Unterschreiten eines Grenzwerts, der in der Speichereinrichtung 20 hinterlegt sein kann, ein Warnsignal ausgegeben wird.

Wie Figur 1 zeigt, ist die Anzeigeeinrichtung 19 signalübertragend, insbesondere leitungslos mit der Steuereinrichtung 8 verbunden.

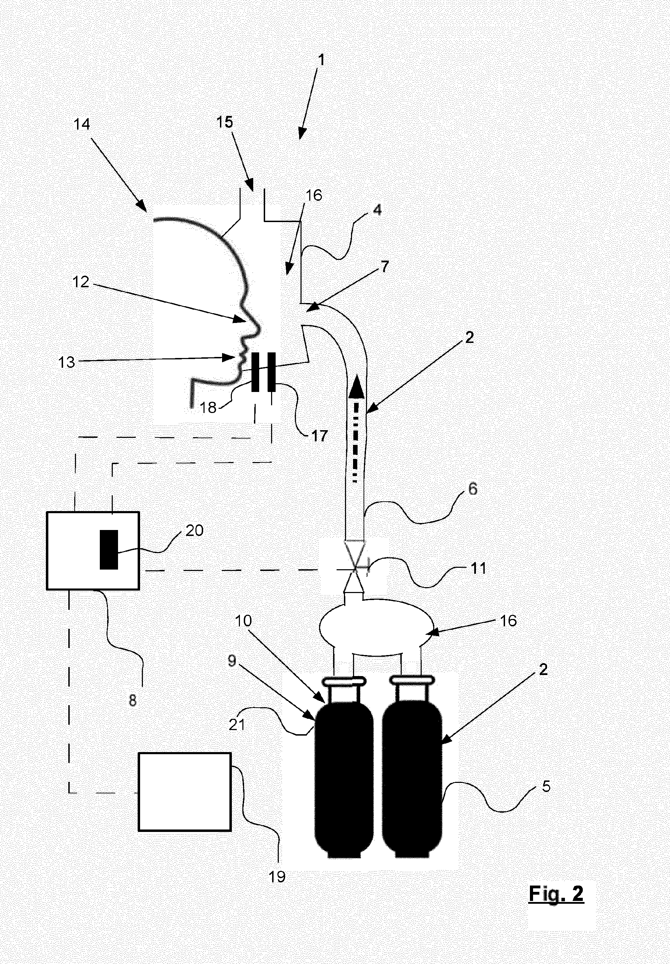

Gemäß einer weiteren Ausgestaltung, die in Figur 2 gezeigt ist, kann ein weiteres Gas 9 beziehungsweise ein weiteres Gasgemisch 10 vorgesehen sein, das in einem weiteren Behälter 21 gespeichert ist. Beispielsweise kann das Gasgemisch 2 im Behälter 5 Sauerstoff und Kohlenstoffdioxid umfassen, während das weitere Gasgemisch 10 Ozon und Sauerstoff umfassen kann.

Die jeweils in den Behältern 5 und 21 enthaltenen Gase 3, 9 beziehungsweise Gasgemische 2, 10 werden in dem Mischraum 16, der in Fluidrichtung im Wesentlichen unmittelbar nach den Behältern 5 und 21 angeordnet ist, zusammengeführt und miteinander gemischt. Dieser Mischraum 16 ist in Figur 2 dargestellt.

Außerdem umfasst die Vorrichtung 1 gemäß der Ausgestaltung in Figur 2 einen Lufteinlass 15, der an dem Inhalationsteil 4 angeordnet ist. Mittels dieses Lufteinlasses 15 ist eine Umgebung fluidleitend mit dem Inhalationsteil 4 verbunden. Mit anderen Worten kann Umgebungsluft mittels des Lufteinlasses 15 in das Inhalationsteil 4 einströmen sowie beispielsweise das Gasgemisch 2 oder von der Person 14 ausgeatmete Luft aus dem Inhalationsteil 4 in die Umgebung hinaus ausströmen.

### Bezugszeichenliste

- 1: Vorrichtung
- 2: Gasgemisch
- 3: Ozon
- 4: Inhalationsteil
- 5: Behälter
- 6: Leitungselement
- 7: Öffnung
- 8: Steuereinrichtung
- 9: weiteres Gas
- 10: weiteres Gasgemisch
- 11: Ventileinrichtung
- 12: Mundbereich
- 13: Nasenbereich
- 14: Person
- 15: Lufteinlass
- 16: Mischraum
- 17: Drucksensor
- 18: Konzentrationssensor
- 19: Anzeigeeinrichtung
- 20: Speichereinrichtung
- 21: weiterer Behälter

## Patentansprüche

1. Vorrichtung (1) zum Inhalieren eines Gasgemisches mit zumindest einem Anteil
Ozon, umfassend
ein Inhalationsteil (4)
einen Behälter (5) zum Speichern von Ozon (3) oder des Gasgemisches (2) ein Leitungselement (6), mittels welchem der Behälter (5) fluidleitend mit dem Inhalationsteil (4) verbunden ist,
wobei das Inhalationsteil (4) wenigstens eine Öffnung (7) aufweist, aus der das Gasgemisch (2) ausströmen kann, **dadurch gekennzeichnet, dass** das Gasgemisch (2) in dem Behälter (5) gasförmig vorliegt.

2. Vorrichtung (1) nach Anspruch 1, **gekennzeichnet durch** eine Steuereinrichtung, (8) mittels derer eine Zusammensetzung des Gasgemisches (2), insbesondere dessen Anteil an Ozon (3), durch Mischung mit einem weiteren Gas (9) oder Gasgemisch (10) eingestellt werden kann.

3. Vorrichtung (1) nach Anspruch 1 oder 2, **gekennzeichnet durch** eine Ventileinrichtung (11), mittels welcher ein Durchfluss des Gasgemisches (2) unterbrechbar und/oder regulierbar ist.

4. Vorrichtung (1) nach Anspruch 2 und 3, **dadurch gekennzeichnet, dass** die Ventileinrichtung (11) mittels der Steuereinrichtung (8) steuerbar ist.

5. Vorrichtung (1) nach einem der vorangehenden Ansprüche, **dadurch gekennzeichnet, dass** das Inhalationsteil (4) eine Maske aus einem fluidundurchlässigen Material ist, welche zumindest einen Mundbereich (12) und Nasenbereich (13) einer die Maske tragenden Person (14) umschließt.

6. Vorrichtung (1) nach einem der vorangehenden Ansprüche, **dadurch gekennzeichnet, dass** das Inhalationsteil (4) eine Maske aus einem fluiddurchlässigen Material ist.

7. Vorrichtung (1) nach Anspruch 6, **dadurch gekennzeichnet, dass** das Inhalationsteil (4) einen Lufteinlass (15) aufweist.

8. Vorrichtung (1) nach einem der vorangehenden Ansprüche, **gekennzeichnet durch** einen Mischraum (16) zum Mischen des aus dem Behälter (5) austretenden Ozons (3) oder Gasgemisches (2) mit dem weiteren Gas (9) oder Gasgemisch (10) , wobei der Mischraum an dem Behälter (5) oder in dem Leitungselement (6) oder in dem Inhalationsteil (4) angeordnet ist.

9. Vorrichtung (1) nach einem der vorangehenden Ansprüche, **gekennzeichnet durch** einen Drucksensor (17), mittels welchem ein Druck des Gasgemisches (2) ermittelbar ist.

10. Vorrichtung (1) nach einem der vorangehenden Ansprüche, **gekennzeichnet durch** einen Konzentrationssensor (18), mittels welchem die Zusammensetzung des Gasgemisches (2), insbesondere in dem Inhalationsteil (4), ermittelbar ist.

11. Vorrichtung (1) nach Anspruch 10, **dadurch gekennzeichnet, dass** ein von dem Konzentrationssensor (18) ermittelter Wert mittels einer Anzeigeeinrichtung (19) darstellbar ist.
